(19) **Europäisches Patentamt**

**European Patent Office**

**Office européen des brevets**

(11) **EP 1 078 957 B1**

(12) **EUROPÄISCHE PATENTSCHRIFT**

(45) Veröffentlichungstag und Bekanntmachung des Hinweises auf die Patenterteilung:
**26.06.2002   Patentblatt 2002/26**

(51) Int Cl.⁷: **C09C 1/36**, C09C 3/12, C08K 9/06

(21) Anmeldenummer: **00107734.6**

(22) Anmeldetag: **11.04.2000**

(54) **Oberflächenmodifiziertes Titandioxid**

Surface modified titanium dioxide

Dioxide de titanium à surface modifiée

(84) Benannte Vertragsstaaten:
**AT BE CH CY DE DK ES FI FR GB GR IE IT LI LU MC NL PT SE**

(30) Priorität: **29.06.1999   DE 19929845**

(43) Veröffentlichungstag der Anmeldung:
**28.02.2001   Patentblatt 2001/09**

(73) Patentinhaber: **Degussa AG**
**40474 Düsseldorf (DE)**

(72) Erfinder:
• **Deller, Klaus, Dr.**
  **63515 Hainburg (DE)**
• **Kerner, Dieter, Dr.**
  **63450 Hanau (DE)**
• **Meyer, Jürgen, Dr.**
  **63811 Stockstadt/Main (DE)**

(56) Entgegenhaltungen:
EP-A- 0 808 880          WO-A-00/31178
WO-A-98/53004          DE-A- 3 707 226

EP 1 078 957 B1

**Beschreibung**

Die Erfindung betrifft ein oberflächenmodifiziertes, pyrogen hergestelltes Titandioxid, ein Verfahren zu seiner Herstellung sowie seine Verwendung.

Es ist bekannt, daß hochdisperses pyrogen hergestelltes Titandioxid (zum Beispiel Titandioxid P 25 S der Degussa-Hüls AG) wegen seiner niedrigen mittleren Primärpartikelgröße (21 nm) und der aus dem pyrogenen Herstellungsprozess resultierenden Aggregat- und Agglomeratstruktur vorteilhafte Eigenschaften in kosmetischen Sonnenschutzmittelformulierungen als UV-A und UV-B Blocker hat. Desweiteren ist bekannt, daß sich ein steigender Dispergierungsgrad des Titandioxides vorteilhaft auf die UV-Schutzwirkung auswirkt.

Die hydrophile Oberfläche des pyrogen hergestelltes Titandioxides macht den Einsatz hoher Scherkräfte erforderlich, um einen hohen Dispergierungsgrad vorallem in oleophilen Formulierungen zu erzielen. Es sind deshalb bereits mit Alkylsilylgruppen oberflächenmodifizierte pyrogen Titandioxide (Titandioxid T 805 der Degussa-Hüls Aktiengesellschaft) bekannt, die neben der verbesserten Dispergierbarkeit in oleophielen Formulierungen bereits bei niedrigen Scherenergien, eine verbesserte UV-Blockung und eine verminderte photochemische Aktivität des pyrogenen Titandioxides bedingen. (Technical Information TI 1176 "Highly dispersed Titanium Dioxide for sunblock preparations").

Nachteilig an den bekannten oberflächenmodifizierten pyrogenen Titandioxiden ist die durch den ausschliesslich oleophilen Oberflächencharakter bedingte niedrige Wechselwirkung mit hydrophilen Bestandteilen der kosmetischen Sonnenschutzformulierungen.

Aufgabe der Erfindung ist die Entwicklung eines pyrogen hergestellten Titandioxides, dessen Oberflächenmodifizierung ihm sowohl oleophilen als auch hydrophilen Charakter verleiht, und das eine gute Dispergierbarkeit in hydrophilen und oleophilen Medien besitzt.

Gegenstand der Erfindung ist oberflächenmodifiziertes, pyrogen hergestelltes Titandioxid, welches dadurch gekennzeichnet ist, daß es mit mindestens einem ammoniumfunktionellen Silan behandelt ist und die physikalisch-chemischen Kenndaten

| | |
|---|---|
| BET-Oberfläche | 15 bis 100 m$^2$/g |
| Schüttdichte | 150 bis 700 g/l |
| pH-Wert | 2,5 bis 8,5 |
| Trockenverlust | 0,1 bis 2,0 Gew.-% |
| Glühverlust | 0,3 bis 13,0 Gew.-% |
| Kohlenstoffgehalt | 0,3 bis 12,0 Gew.-% |
| Methanolbenetzbarkeit | 0 bis 50 Gew.-% |

aufweist.

In einer Ausführungsform der Erfindung kann der Gegenstand der Erfindung ein oberflächenmodifiziertes pyrogen hergestellten Titandioxid sein welches, dadurch gekennzeichnet ist, daß zur Oberflächenmodifizierung ein ammoniumfunktionelles Silan der allgemeinen Formel

$$(RO)_{3-n+m}\,SiR_n^1\{(CH2)_y[NR^2R^3R^4]^+X^-\}_m$$

n = 0-2; m= 1-2 mit n+m<3; y=1-5; X = F, Cl, Br, I R = Alkyl (C1-C5); R$^1$=Alkyl (C1-C18), Aryl; R$^2$=H, Alkyl, Aryl, Benzyl;

R$^4$ = H, Alkyl, Aryl, -C$_2$H$_4$NR$^5_2$ mit R$^5$=Alkyl

oder eines der folgend genannten Silane

$$(RO)_3SiC_3H_6[N_4C_6H_{12}]^+X^-$$

R = Alkyl (C1-C5); X=Cl, Br, I

$$(RO)_3SiC_3H_6[pyridinyl]^+X^-$$

R = Alkyl (C1-C5); X=Cl, Br, I

$$(RO)_3SiC_3H_6[OCH_2CH(OH)CH_2[N(CH_3)_3]^+X^-$$

R = Alkyl (C1-C5); X=Cl, Br, I oder eines der nachfolgenden Silane

$$2Cl^- \quad C_6H_{12}N_4^{2+}[-C_3H_6-Si(OC_2H_5)_3]_2$$

$$3Cl^- \quad C_6H_{12}N_4^{3+}[-C_3H_6-Si(OC_2H_5)_3]_3$$

$$Cl^- \quad (CH_3)_2N-C_2H_4-N^+-(CH_3)2C3H_6-Si(OC_2H_5)_3$$

$$2Br^- \quad CH_2[-^+N(CH_3)_2-C_3H_6-Si(OC_2H_5)_3]_2$$

$$2J^- \quad [-CH_2{}^+NH(CH_3)-C_3H_6-Si(OC_2H_5)_3]_2$$

oder ein Gemisch zwei oder mehrerer der vorgenannten Silane eingesetzt wird.
Als Silane könnte zum Beispiel eingesetzt werden:

$$(C_2H_5O)_3SiC_3H_6[N(CH_3)_3]^+X^-$$

$$(C_2H_5O)_3SiC_3H_6[N(CH_2)_2(C_{18}H_{37})]^+X^-$$

$$Cl^- \quad (CH_3)_3{}^+N-C_3H_6-Si(OC_2H_5)_3$$

$$J^- \quad (C_6H_5)(CH_3)_2{}^+N-C_3H_6-Si(OC_2H_5)_3$$

$$Br^- \quad (C_6H_5-CH_2)(CH_3)_2{}^+N-C_3H_6-Si(OC_2H_5)_3$$

$$Cl^- \quad (C_{12}H_{25})(CH_3)_2{}^+N-C_3H_6-Si(OC_2H_5)_3$$

$$Br^- \quad (C_{12}H_{25})(CH_3)_2{}^+N-C_3H_6-Si(OC_2H_5)_3$$

$$J^- \quad (C_{12}H_{25})(CH_3)_2{}^+N-C_3H_6-Si(OC_2H_5)_3$$

$$Cl^- \quad (C_{16}H_{33})(CH_3)_2{}^+N-C_3H_6-Si(OC_2H_5)_3$$

$$Cl^- \quad (C_2H_5)_3{}^+N-C_3H_6-Si(OC_2H_5)_3$$

$$J^- \quad (C_2H_5)_3{}^+N-C_2H_4-Si(OC_2H_5)_3$$

$$J^- \quad (C_2H_5)_3{}^+N-C_3H_6-Si(OC_2H_5)_3$$

$$Cl^- \qquad (C_4H_9)_3{}^+N-C_3H_6-Si(OC_2H_5)_3$$

$$Br^- \qquad (C_4H_9)_3{}^+N-C_3H_6-Si(OC_2H_5)_3$$

Ein weiterer Gegenstand der Erfindung ist ein Verfahren zur Herstellung des oberflächenmodifizierten, pyrogen hergestellten Titandioxides, welches dadurch gekennzeichnet ist, daß man das pyrogen hergestellte Titandioxid in einem Mischaggregat unter intensivem Mischen, gegebenenfalls in Gegenwart von Wasser oder verdünnter Säure, mit dem Silan, dem Silangemisch oder einer Lösung des Silanes oder Silangemisches in Ethanol besprüht, und nach ca. 10 - 30 Minuten Nachrühren bei einer Temperatur von 100 - 400°C zwischen 0,5 und 6 h tempert. Die Temperung kann unter einer Schtuzgasatmosphäre, zum Beispiel $N_2$, durchgeführt werden.

Als pyrogen hergestelltes Titandioxid kann man ein Titandioxid verwenden, wie es in Ullmanns Enzyklopädie der technischen Chemie 4. Auflage Band 21 (1982) Seiten 464 - 465 beschrieben wird.

Das erfindungsgemäße oberflächenmodifizierte, pyrogen hergestellte Titandioxid kann im Kosmetikbereich, insbesondere als Sonnenschutzmittel, weiterhin in Tonerpulvern, Farben und Lacken, Silikonkautschuk, als Schleif- und Poliermittel zum Beispiel im CMP-Bereich, eingesetzt werden.

Im Kosmetikbereich insbesondere in Sonnenschutzformulierungen zeigt das erfindungsgemäße oberflächenmodifizierte pyrogen hergestellte Titandioxid folgende Vorteile:

- verbesserte Absorption der Strahlung im Bereich von 300-500 nm

- gute Dispergierbarkeit in oleophilen und hydrophilen Formulierungen

**Beispiele**

Als Titandioxid wird das auf pyrogenem Wege hergestellte Titandioxid P 25 eingesetzt. Das Titandioxid P 25 ist bekannt aus der Schriftenreihe Pigmente No. 56 "Hochdisperse Metalloxide nach dem Aerosilverfahren", 4. Auflage Februar 1989 Degussa AG.

Das Titandioxid P 25 weist die folgenden physikalisch-chemischen Kenndaten auf:

| | **Titandioxid P 25** |
|---|---|
| CAS-Reg. Nummer | 13463-67-7 |
| Verhalten gegenüber Wasser | hydrophil |
| Aussehen | lockeres weißes Pulver |
| Oberfläche nach BET[1]) $\qquad$ $m^2/g$ | 50 + 15 |
| Mittlere Größe der Primärteilchen $\qquad$ nm | 21 |
| Stampfdichte[2]) $\qquad$ g/l | ca. 100 |
| Spezifisches Gewicht[10]) $\qquad$ g/ml | ca. 3,7 |
| Trocknungsverlust[3]) bei Verlassen des Lieferwerkes (2Stunden bei 105°C) $\qquad$ % | < 1,5 |
| Glühverlust[4])[7]) (2 Stunden bei 1000°C) | < 2 |
| pH-Wert[5]) (in 4%iger wäßriger Dispersion) | 3-4 |
| $SiO_2$[8]) | < 0,2 |
| $Al_2O_3$[8]) | < 0,3 |

1) nach DIN 66131

2) nach DIN ISO 787/XI, JIS K 5101/18 (nicht gesiebt)

3) nach DIN ISO 787/II, ASTM D 280, JIS K 5101/21

4) nach DIN 55921, ASTM D 1208, JIS K 5101/23

5) nach DIN ISO 787/IX;ASTM D 1208 JIS K 5101/24

7) bezogen auf die 2 Stunden bei 105°C getrocknete Substanz

8) bezogen auf die 2 Stunden bei 1000°C geglühte Substanz

10) bestimmt mit dem Luftvergleichspyknometer

(fortgesetzt)

| | Titandioxid P 25 |
|---|---|
| Fe2O38) | < 0,01 |
| TiO28) | > 99,5 |
| ZrO28) | - |
| HfO28) | - |
| HCl8)9) | < 0,3 |
| Siebrückstand6) (nach Mocker, 45 μm)       % | < 0,05 |

6) nach DIN ISO 787/XVIII; JIS K 5101/20

8) bezogen auf die 2 Stunden bei 1000°C geglühte Substanz

9)HCl-Gehalt ist Bestandteil des Glühverlustes

## Herstellung - Allgemeine Durchführung

$TiO_2$ P25 wird in einem Mischer vorgelegt und unter Mischen mit dem Silan besprüht. Das Silan kann in einem Lösungsmittel, zum Beispiel Ethanol, gelöst sein. Nach beendetem Aufsprühen wird noch 10 - 30 Minuten nachgemischt und anschließend bei einer Temperatur von 100 - 400°C über einen Zeitraum vom 0,5 - 6 Stunden getempert. Das Besprühen und/oder die Temperung kann in einer Schutzgasatmosphäre, wie zum Beispiel unter Stickstoff, durchgeführt werden.

## Herstellung der beschriebenen Beispiele

| Beispiel | Silan | Silanmenge [g/100g P25] | Ethanolmenge [g/100g P25] | Temperzeit [h] | Temperatur [°C] |
|---|---|---|---|---|---|
| 1 | Si 270/75 | 5 | 10 | 2 | 120 |
| 2 | Si 270/75 | 7,5 | 0 | 2 | 120 |
| 3 | Si 270/75 | 10 | 20 | 2 | 120 |
| 4 | Si 275/75 | 5 | 10 | 2 | 120 |
| 5 | Si 275/75 | 7,5 | 0 | 2 | 120 |
| 6 | Si 275/75 | 10 | 20 | 2 | 120 |

## Eingesetzte Silane

$$Si\ 270/75 = Cl^{-}(CH_3)_3N^{+} - C_3H_6-Si(OC_2H_5)_3\ (75\ \%ige\ L\ddot{o}sung\ in\ Ethanol)$$

$$Si\ 275/75 = Cl^{-}(C18H_{37})\ (CH_3)_2N^{+} - C_3H_6-Si(OC_2H_5)_3\ (75\ \%ige\ L\ddot{o}sung\ in\ Ethanol)$$

## 1. Physikalisch-chemische Daten

| Beispiel | Silan | Stampf-dichte (g/l) | Kohlen-stoffgehalt (%) | PH-Wert | Trocknungs-verlust (%) | Glüh-verlust (%) | CH$_3$OH-Benetzbarkeit Gew.-% |
|---|---|---|---|---|---|---|---|
| 1 | Si 270/75 | 225 | 1,3 | 3,7 | 0,5 | 2,9 | 0 % |
| 2 | Si 270/75 | 282 | 1,6 | 3,5 | 0,8 | 3,6 | 0 % |
| 3 | Si 270/75 | 397 | 2,1 | 3,6 | 0,4 | 4,6 | 0 % |
| 4 | Si 275/70 | 215 | 2,1 | 3,6 | 0,8 | 3,4 | 0 % |
| 5 | Si 275/70 | 368 | 3,2 | 3,3 | 1,0 | 4,9 | 20 % |
| 6 | Si 275/70 | 384 | 3,5 | 3,3 | 0,8 | 5,2 | 10 % |

Die Produkte zeigen hydro- und oleophilen Charakter und können in kosmetischenFormulierungen vorteilhaft eingesetzt wertden.

EP 1 078 957 B1

Anwendungstechnische Prüfergebnisse

Die gemessenen UV/VIS-Spektren von angefertigten Sonnenschutzpasten zeigen gute Absorptionen im UVA- und UVB-Bereich.

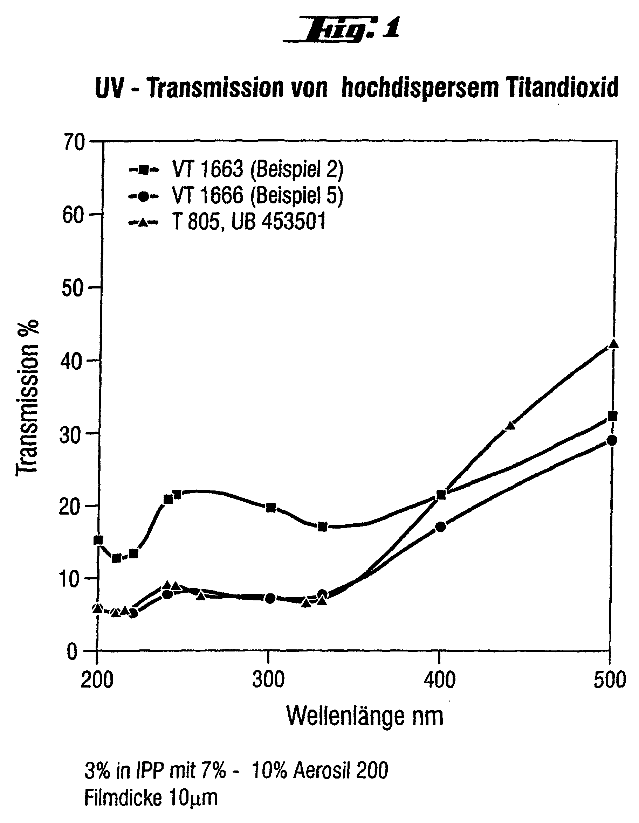

Der Vergleich zu dem bekannten Produkt T 805 ist in der Figur 1 dargestellt.

Diese Ergebnisse zeigen, daß die erfindungsgemäßen Titandioxide sehr gut in Sonnenschutzformulierungen eingesetzt werden können.

So wird vorallem in dem Bereich von 300 bis 500 nm eine bessere Absorption der Strahlung erreicht.

Das Produkt T 805 ist ein pyrogen hergestelltes Titandioxid, das mit einem Trialkoxyoctylsilan behandelt wurde.

**Patentansprüche**

1. Oberflächenmodifiziertes, pyrogen hergestelltes Titandioxid, **dadurch gekennzeichnet, daß** es mit mindestens einem ammoniumfunktionellen Silan behandelt ist und die physikalisch-chemischen Kenndaten:

| BET-Oberfläche | 15 bis 100 m$^2$/g |
|---|---|
| Schüttdichte | 150 bis 700 g/l |
| pH-Wert | 2,5 bis 8,5 |
| Trockenverlust | 0,1 bis 2,0 Gew.-% |
| Glühverlust | 0,3 bis 13,0 Gew.-% |
| Kohlenstoffgehalt | 0,3 bis 12,0 Gew.-% |
| Methanolbenetzbarkeit | 0 bis 50 Gew.-% |

aufweist.

2. Verfahren zur Herstellung der oberflächenmodifizierten, pyrogen hergestellten Titandioxide gemäß Anspruch 1, **dadurch gekennzeichnet, daß** man das pyrogen hergestellte Titandioxid in einem Mischaggregat unter intensivem Mischen gegebenenfalls in Gegenwart von Wasser oder verdünnter Säure mit dem Silan, dem Silangemisch oder einer Lösung des Silanes oder Silangemisches in Ethanol besprüht, und nach ca. 10 - 30 min Nachrühren bei Temperaturen von 100 - 400°C zwischen 0,5 und 6 h tempert.

3. Verwendung des oberflächenmodifizierten, pyrogen hergestellten Titandioxides gemäß Anspruch 1 in Kosmetika, in Sonnenschutzmitteln, in Tonerpulvern, in Farben und Lacken, in Silikonkautschuk, als Schleif- und Poliermittel, zum Beispiel im CMP-Bereich.

**Claims**

1. Surface-modified, pyrogenically produced titanium dioxide, **characterised in that** it is treated with at least one ammonium-functional silane and has the following physicochemical data:

| BET surface area | 15 to 100 m$^2$/g |
|---|---|
| Bulk density | 150 to 700 g/1 |
| pH value | 2.5 to 8.5 |
| Loss on drying | 0.1 to 2.0 wt.% |
| Loss on ignition | 0.3 to 13.0 wt.% |
| Carbon content | 0.3 to 12.0 wt.% |
| Methanol wettability | 0 to 50 wt.% |

2. Process for the production of the surface-modified, pyrogenically produced titanium dioxides according to claim 1, **characterised in that** the pyrogenically produced titanium dioxide, in a mixing unit with intensive mixing, optionally in the presence of water or dilute acid, is sprayed with the silane, with the silane mixture or with a solution of the silane or silane mixture in ethanol, and then further stirred for approximately 10 to 30 minutes and subsequently tempered for between 0.5 and 6 hours at a temperature of 100 to 400°C.

3. Use of the surface-modified, pyrogenically produced titanium dioxide according to claim 1 in cosmetics, in sunblocks, in toner powders, in paints and varnishes, in silicone rubber, as abrasives and polishes, for example, in the field of CMP.

**Revendications**

1. Dioxyde de titane produit par voie pyrogénique dont les surfaces ont été modifiées,
   **caractérisé en ce qu'**
   il est traité par au moins un silane fonctionnalisé par un ammonium et qui possède les caractéristiques physico-chimiques :

   | | |
   |---|---|
   | Surface BET | de 15 à 100 m$^2$/g |
   | Densité apparente | de 150 à 700 g/l |
   | Valeur du pH | de 2,5 à 8,5 |
   | Perte à la dessication | de 0,1 à 2,0 % en poids |
   | Perte à la calcination | de 0,3 à 13,0 % en poids |
   | Teneur en carbone | de 0,3 à 12,0 % en poids |
   | Mouillabilité au méthanol | de 0 à 50 % en poids |

2. Procédé de préparation des dioxydes de titane produits par voie pyrogénique dont les surfaces ont été modifiées conformément à la revendication 1,
   **caractérisé en ce qu'**
   on pulvérise le dioxyde de titane produit par voie pyrogénique dans un appareillage de mélange tout en mélangeant intensément le cas échéant en présence d'eau ou d'acide dilué, avec le silane, le mélange de silanes ou avec une solution du silane ou du mélange de silanes dans l'éthanol, et on recuit après environ 10 à 30 minutes de poursuite de l'agitation à des températures allant de 100 à 400°C pendant entre 0,5 et 6 heures.

3. Utilisation du dioxyde de titane produit par voie pyrogénique dont les surfaces ont été modifiées conformément à la revendication 1, dans des cosmétiques, dans des produits de protection solaire, dans des poudres de toner, dans des colorants et des laques, dans le caoutchouc silicone, comme agents abrasif et de polissage, par exemple dans le domaine CMP.

## *Fig. 1*

### UV - Transmission von hochdispersem Titandioxid

- ■ - VT 1663 (Beispiel 2)
- ● - VT 1666 (Beispiel 5)
- ▲ - T 805, UB 453501

Transmission %

Wellenlänge nm

3% in IPP mit 7% - 10% Aerosil 200
Filmdicke 10μm